# EUROPEAN PATENT APPLICATION

(11) **EP 1 997 831 A1**
(43) Date of publication of application: **03.12.2008**
(21) Application number: 07711103.7
(22) Date of filing: 28.02.2007
(51) Int. Cl.: C07K 16/10

(54) **CHIMERIC VACCINE ANTIGENS AGAINST THE AVIAN INFLUENZA VIRUS**

(30) Priority: 28.02.2006 CU 512006
(71) Applicant: CENTRO DE INGENIERIA GENETICA Y BIOTECNOLOGIA, Ciudad de La Habana 10600 (CU)
(72) Inventor: SÁNCHEZ RAMOS, Oliberto, Ciudad Habana 10400 (CU); TOLEDO ALONSO, Jorge, Roberto, Ciudad Habana 10400 (CU); DÍAZ ARCHER, Damarys, Ciudad Habana 11600 (CU); FIGUEROA BAILE, Nancy, Elena, Ciudad Habana 11 600 (CU); RODRÍGUEZ MOLTÓ, María, Pilar, Ciudad De La Habana 11 600 (CU); BORROTO NORDELO, Carlos, Guillermo, Ciudad Habana 11600 (CU)
(74) Representative: Hatzmann, Martin
(86) International application number: PCT/CU2007/000009
(87) International publication number: WO 2007/098718

(57) **Abstract**

The present invention describes chimeric vaccine antigens against Avian Influenza virus (AI). Such vaccine antigens are based on virus subunits that are coupled to protein co-stimulatory molecules that enhance both cellular and humoral arms of the immune response. Chimeric antigens can be produced on expression systems that guarantee a correct tridimensional folding of chimeric molecules constituting the basis of the present invention. The vaccine compositions containing such antigens induce an early and strong immune response on vaccinated birds and mammalians, stimulating high titers of antibodies inhibitory of the hemagglutination and a strong cellular response specific against the virus antigen. The chimeric antigens, as well as the resulting vaccine compositions are applicable to human and animal health, as vaccines for preventive use.

## Description

### Technical Field

The present invention is related with the human and veterinary medicine field, in particular, with new chimeric antigens, which comprise the virus subunits of Avian Influenza virus, coupled to co-stimulatory molecules that enhance both the humoral and cellular immune system, developing in both birds and mammalians a strong and early immune response against such virus.

### Previous art

Avian Influenza (Al) is a respiratory disease distributed worldwide. This disease, which is highly contagious, can affect chickens, turkeys, ducks, gooses, guinea hens, as well as a wide variety of domestic and wild birds. There exists the possibility that all avian species are susceptible to infection, being the migratory aquatic species, the principal natural reservoir of the virus that causes this disease.

The avian influenza virus infection in domestic poultry provokes two types of disease that are distinguished according its high or low level of virulence. The "low pathogenic" form can pass unnoticed and generally produces mild symptoms only (such as bristly feathers and a decreasing of eggs production). Nevertheless, the highly pathogenic form is faster spread among poultry. This form can cause diseases that attack several internal organs having a death rate, which can reach until 90-100% in a period of 48 hours.

The virus responsible of Avian Influenza pertains to the Ortomixoviridae family. Influenza viruses are divided in A, B and C types based on its antigenic differences. Its genetic material is ribonucleic acid (ARN) of negative polarity, segmented. Influenza viruses A and B, have 8 segments, while C influenza virus has 7 segments only. The fact that the virus genome will be segmented favors genetic recombination, originating a virus with characteristics different from the original (Capua; Alexander (2004) Avian Influenza: Recent Developments. Avian Pathol. 33:393-404).

The virus envelope contains two main glycoproteins, hemagglutinin (HA) and neuraminidase (NA). HA protein is the binding protein to the cellular receptor, it mediates the fusion of the virus envelope with the cellular membrane, important for the penetration of the virus to the target cell. This protein induces a response of neutralizing antibodies. Mutations on the HA protein cause antigenic variants of greater or smaller extension. Protein NA cuts the sialic acid of the host's cells, favoring the virus release from the cells, degrades the mucus and makes easy the access of virus to the tissue. NA of Avian Influenza virus also suffers antigenic changes.

Normally, the viruses of avian influenza do not infect others species apart from fowl and pigs. The first case of human infection due to an avian influenza virus was documented in Hong Kong, in 1997, when H5N1 strain caused an acute respiratory disease to 18 persons, from which six died. Infection in those individual coincided with an epidemic of avian influenza highly pathogenic on the avian population in Hong Kong, produced by the same strain. On February, 2003, in Hong Kong, a new alert was produced, when an outbreak of avian influenza H5N1 caused one death among members of a family which have been traveled to South China recently. Another child of the family died during such visit but the cause of his death is unknown.

Recently, another two viral strains of Al have caused disease in humans. In Hong Kong, in 1999, two mild cases of AI H9N2 were produced in children, and at the middle of December, 2003, another case was reported. H9N2 subtype in fowl, commonly, is not highly pathogenic. Nevertheless, an H9N2 avian influenza virus highly pathogenic, which started on February, 2003 in the Netherlands, caused the death of a veterinarian two months later, and mild disease in another 83 persons.

Since 1997 until now, outbreaks of avian influenza capable of infecting humans have been more frequent, and the virus has spread through a great number of countries from Asia and Europe. Until the moment, the H5N1 strain has been the main causative agent of influenza in humans. Until October, 2005, 200 persons infected by H5N1 have been reported, with a death rate of 55% approximately. Thirteen countries from Asia and Europe have been affected, and more than 120 million of birds have died or been situated on quarantine.

In general, each outbreak of AI, leads to the sacrifice of hundred millions of birds and to the adoption of sanitary control rules, which as a whole means big economic losses.

During an outbreak of Avian Influenza among poultry, exists the risk that persons that had contact with infected birds or surfaces contaminated with secretions and excretions of such birds get contagious. Spreading of infection between birds increases the opportunities of direct infection of humans. If more persons acquire the infection, with the time increases also the risk, if persons infected by avian and human influenza strains, could serve as "mixture recipients" for the appearance of a new subtype. This new subtype, with enough genes of human influenza virus, could be easily transmitted from person to person, and will be transformed on a pandemic strain completely transmissible, as the variant of H1N1 which in 1918 generated a pandemic known as "Spanish Influenza", where among 25 and 50 millions of persons died.

An immediate priority is to stop the additional dissemination of the pandemic between fowl population. This strategy is effective for the reduction of opportunities of human exposition to the virus. Vaccination of persons with high risk of exposition to infected birds, using the effective vaccines against the strains of influenza virus now in currency, can reduce the probability to get human infection by avian and human influenza strains, and in this manner diminishing the risk to produce a genetic exchange between both virus strains.

Nowadays, for the prevention of the disease not only in humans but also in birds, procedures for the production of vaccines against influenza are based on the propagation of virus on chicken's embryo. Subsequently, viruses produced by this way are chemically inactivated and semi-purified. Nevertheless, such technology is unable of answering to a possible pandemic crisis. Through this procedure, the development and production of a vaccine takes several months.

After the identification of a potential strain, its absorption is required with a highly productive strain to obtain the adequate growing properties. Even, it is more important that H5 strains responsible of recent epizootic, are associated to a several infection cases in humans resulting lethal in chicken embryos that are used in vaccines production. The production of these vaccines additionally implies the manipulation of pathogenic strains. Due to this is necessary the work under safety conditions BL3 with the consequent enhancement of the process and the difficulties to carry out a scale up in case of crisis.

It has been demonstrated that persons with acute allergies to egg can suffer immediate reactions of hypersensitivity, due to the residual egg's protein on the vacunal preparations against influenza. In 1976, vaccine against swine influenza was associated to an increase in the frequency of Guillain-Barre Syndrome (Schonberger et al. (1979) Syndrome following vaccination in the National Immunization Program, United States, 1977-1977, Am. J. Epidemio, 110-105-23). Until now, has been not observed an increase in the occurrence of this disease with the subsequent vaccine preparations from other strains.

Production of virus based on culture cells had emerged as an attractive alternative to replace the production systems in chicken embryos. Such strategy implies the production of the influenza virus in culture cells, followed by a virus purification step. This procedure has the advantages that: 1) Culture cells are easy to manipulate and scaled on a short period of time, 2) Vaccines of influenza produce on these systems have been evaluated on Phase I and Phase II clinical trials, and it has been demonstrated that are safe and at least as effective as those produced on chicken embryos (Brands et al. (1999) Influvac: a sale Madin Darby Canine Kidney (MDCK) cell culture based on influenza vaccine. Dev Biol Stand. 98:93-100; Percheson et al. (1999) A Phase I, randomized controlled clinical trial to study the reactogenicity and immunogenicity of a new split influenza B virus vaccines grown in mammalian cells or embryonated chicken eggs. J. Virol. 72:4472-7). Nevertheless, this strategy still has as a limitation that is required reabsorbed virus which allow high yields. The process could introduce also specific mutations of the cellular line in virus genes, which in principle, could lead to the selection of variants characterized by structural and antigenic changes on HA protein, resulting potentially on vaccines less effective. (Meiklejohn et al. (1987) Antigen drift and efficacy of influenza virus vaccines. J Infect Dis. 138:618-24;Robertson et al. (1985) Alterations in the hemagglutinin associated with adaptation of influenza B virus to growth in eggs. Virology 143: 166-74; Schild et al. (1983) Evidence for host-cell selection of influenza virus antigenic variants. Nature 303:706-9). Among additional limitations are included: 1) Production and Manipulation of pathogenical virus demands of facilities of high contention; 2) In general the production systems based on cellular culture are expensive and technically exigent.

Protection against influenza virus is the result of the immune response against HA protein, from which exists 15 of different subtype, and in a lesser measure against NA protein, from which exists 9 subtypes reported (Suarez, Schultz (2000) Immunology of avian influenza virus: a review. Dev. Comp. Immunol. 24:269-283;Swayne, Halvorson, (2003) Influenza. In: Saif, Y.M., Barnes, H.J., Fadly, A.M., Glisson, J.R., McDougald, L.R., Swayne, D.E. (Eds). Diseases of Poultry, 11th edn. Iowa State University Press, Ames, IA, pp. 135-160). Immune responses against internal proteins, such as nucleus-protein or the matrix protein are not sufficient to guarantee protection on the field. Practically, protection is provided by the subtype specific of HA included on the vaccine.

Vaccines against Avian Influenza have been produced through the insertion of the HA gene on live virus vectors, and the subsequent use of these recombinants vectors for poultry immunization. Employment of live recombinant virus vectors vaccine has several advantages: 1) They are live vaccines able to induce a response not only humoral but cellular, 2) they can be administrated in small chicken and induce an early protection. For example, a recombinant based on fowl poxvirus can be administrated to birds of one-day age inducing an early protection against Marek disease one week after (Arriola et al. (1999) Experiencias de campo en el uso de vacunos contra influenza aviar. In; Proceedings Curso de Enfermedades Respiratorias de las Aves, Asociación Nacional de Especialistas en Ciencias Avícolas: 3-13). This kind of vaccine makes easy the differentiation between vaccinated and infected birds, because does not induce antibodies against antigens as nucleus protein or the matrix, which are common for Avian Influenza virus. Nevertheless, these vaccines have as a drawback that they may be poorly replicated inducing a partial protective immunity in birds that already have antibodies against the recombinant virus vector, which are capable of neutralizing its vaccine function (Lyschow et al. (2001). Protection of chickens from lethal A avian influenza virus infection by live-virus vaccination with infectious laryngotracheitis virus recombinants expressing the hemagglutinin (H5) gene. Vaccine 19:4249-4259; Swayne et al. (2000) Failure of a recombinant fowl pox virus vaccine containing an avian influenza hemagglutinin gene to provide consistent protection against influenza in chicken pre immunized with a fowl pox vaccine. Avian Dis. 44: 132-137). When recombinant virus vectors are used in young chickens, then the effect of the maternal antibodies can be variable depending on the type of the virus vector employed, and the levels of the transferred maternal antibodies. Another limitation of the use of live recombinant vectors is that the hosts range is restricted (for example, infectious laryngotracheitis is not replicated in gooses), and in consequence these vaccines are restricted to species in which efficacy has been demonstrated.

Generation of effective vaccine candidates against influenza requires significant changes that guarantee a fast response in case of a possible pandemic. Employment of subunit vaccines, based mainly on the use of recombinant HA, constitutes an attractive alternative because this strategy does not involve manipulation of the pathogenic virus, and in consequence, its production does not require of special safety conditions. Antibodies against HA are capable of neutralizing the virus and constitute the basis of the natural immunity to infections with influenza (Clements 1992) "Influenza Vaccines", in Vaccines: New Approaches to Immunological Problems, ed. Ronald W. Ellis, pp. 129-150 (Butterworth-Heinemann, Stoneham, MA). HA is present on the virus envelope in a trimetric form. Each monomer exists as a two chains, HA1 and HA2, coupled by a single disulphide bridge. HA is produced in the host cell as a glycosilated precursor polypeptide, with a molecular mass of about 85 kDa, which is subsequently divided on HA1 and HA2. Antigenic variants on HA molecule are responsible of the influenza outbreaks occurrence and the restricted control of the infection post-immunization.

Until now methods in which the production of recombinant HA, as a potential vaccine of subunits against influenza, is undertaken by baculovirus expression systems have been described (Smith et al. US5858368; Smith et al. US6245532). HA produced on insect cells has been evaluated, in humans (in clinical trials Phase I and Phase II) and in poultry, demonstrating in both cases its safety. However, this kind of vaccine did not result very successful, due mainly to the low titers of neutralizing antibodies that is capable to induce. HA alone, as vacunal antigen, has a very low antigenicity that is reflected on the low titers of antibodies inhibitor of hemagglutination and in a reduced cellular response. Due to the low antigenicity, induction of an effective immune response with this antigen requires of the administration high doses, and frequently, of multiple re-administrations. Due to these inconveniences, and in order to supply the demand of an effective vaccine against Avian Influenza, very high volumes of production are required with the subsequent costs associated to any production system based on culture cells. In addition to these limitations, it is necessary to have in mind logistic complexity and the additional costs associated to the administration of multiple doses in poultry, where the number of animals to be immunized in a single unit can reach tens of thousands.

Therefore, an important problem in the prevention of Avian Influenza is that do not exist, until now, subunit vaccines capable of producing a strong and early immune response after vaccination, that at the same time allow a cost/favorable benefit relation.

### Description of the invention

The present invention solves the problem mentioned before, providing chimeric antigens of vaccine interest against Avian Influenza virus, characterized for containing the extracellular segment of HA from the avian influenza virus envelope and the extra cellular segment of the CD154 molecule. Such chimeric proteins induce an early immune response that protects mammalians and birds from avian influenza virus infections. The obtainment of such vaccine antigens does not require the propagation on eggs, giving as a product a more pure vaccine, with less adverse immune reactions. Besides, does not require a virus inactivation or the extraction of membrane components from the virus, avoids the denaturalization of the antigenic epitopes and others disadvantages relating to the vaccine safety in humans, caused by the residuals of chemical reactives in it. Moreover, an influenza vaccine produced in the absence of egg avoids the heterogeneity that occurs during the adaptation and passage through eggs. This results on a vaccine that adjusts better to epidemic strains of influenza, which leads to a high efficacy.

In an embodiment of the invention it is employed a secretable variant of HA devoid of the transmembrane and the cytoplasmatic domains. Secretable variant of HA is preferably joins to a stimulating sequence of the immune system (molecular adjuvant) which guarantees the obtainment of high titers of neutralizing antibodies against influenza virus.

In a preferred embodiment, the chimeric vaccine antigen is characterized essentially for containing the amino acid sequences of the extra-cellular segment of a CD154 molecule of avian, swine or human origin, identified on the sequence listing with the Sequence Identification (Seq ID.) No. 6, Seq ID. No. 8 and Seq ID. No. 4, respectively.

In an embodiment of the invention, the chimeric vaccine antigen is characterized for containing, essentially, amino acid sequences of the extra cellular segment of HA of virus subtypes H5 (Seq ID. No.2), H7 (Seq ID. No. 9) and H9 (Seq ID. No. 10).

In the context of this invention the term "essentially" refers that the amino acid sequence which is part of the chimeric antigen has a high grade of homology with the numbered sequences, but due to the high variability of this antigen, any amino acid sequence from HA of avian influenza virus can be part of the chimeric antigens object of the present invention. Such chimeric antigens include, but are not restricted to, the prevalent A subtype (H5N1), subtype H9N1, isolates of viral subtype H7, type B that infects humans, as well as the influenza viruses which infect others mammalians and birds species.

For the purpose of the present invention, chimeric antigens could be obtained by recombinant, synthetic or through chemical conjugation way. Sequences coding for HA would be generated by the conventional techniques reverse-transcription and subsequent polymerase chain reaction (PCR). However, in an embodiment of the present invention, the sequences coding for HA are totally synthetic, which guarantees to have the sequence of interest in a period of time considerably short, without the need of working under special safety conditions required for the work with pathogenic virus.

The use of synthetic sequences also allows the optimization of the codon usage, according to the desired expression system. In a similar way, the design of synthetic genes for HA allows the incorporation of punctual mutations that will be translated in changes on the primary structure of the protein, with the aim of increasing its antigenicity.

On a preferred embodiment, chimeric antigen is a heterodimer composed by subunits HA1 and HA2, in which C-terminal sequence is fused a peptide of six histidines that facilitates the purification of the recombinant protein with a purity level higher to 98%. Next, on the C-terminal end of the histidine tail, an spacer peptide composed by four repeated units of Gly4Ser(4G4S) is fused. On the C-terminal sequence of the spacer peptide will be fused the extracellular domain of CD154 molecule as a molecular adjuvant. Peptide 4G4S situated between HA and CD154 molecules, aims to give enough sterical freedom to both molecules in order to get the correct tridimensional conformation.

The chimeric molecule HA-CD154 object of this invention can be a trimer constituted by the non-covalent union of three polypeptides HA-CD154. Nevertheless, in dependence of the expression system used, each monomers HA-CD154 could be divided in two molecules (HA1 and HA2/CD154) generating heterodimers molecules, which can be joined to conform trimers of heterodimers HA1-HA2/CD154. The amino acid sequence correspondent to the CD154 extracellular domain determines trimerization on these molecules. The trimeric structure of the vaccine antigen object of this invention is of great importance for the interaction of the chimeric molecule with the CD40 receptor on the surface of professional antigen presenting cells (APC) of the immune system.

Once released to the bloodstream, the vaccine antigen of this invention specifically interacts with the CD40 receptors on the surface of the APC cell of the immune system. After the interaction with the CD40 receptors, the chimeric molecule HA-CD154 is internalized by the APC cells and is then processed and presented in the context of the Major Histocompatibility Complex (MHC) class I. Simultaneously, the binding of this chimeric protein to dendritic cells (DCs) induces increased levels of secondary signals of activation (CD80 and CD86) and the CCR-7 chemokine receptor on DCs, which leads to the migration of the HA-loaded DCs to the regional lymph nodes. These events induce increases in the levels of the HA-specific CD8+ cytotoxic T lymphocytes in the spleens of the immunized animals.

The chimeric protein HA-CD154 is also capable of interacting specifically with cells B. Initial interaction of HA with the molecule of IgM on the surface of cell B facilitates the interaction of the portion of CD154 of the chimeric molecule with the receptors CD40 on the surface of antigen specific B cells. This stimulation in B cells induces the internalization of the chimeric molecule, its processing and presentation in the MHC class II context. These events lead to the activation of lymphocytes T CD4+ and to the subsequent activation of the T-helper type 2 responses, which induces the immunoglobulin class switching on B cells, its maturation and proliferation.

In a preferred embodiment of the invention, the chimeric vaccine antigen is obtained from milk of genetically modified mammalians. The vaccine antigen, object of this invention will be produced preferably in mammalian milk during the lactation process. With this purpose, production can be done in milk of transgenic animals, in which the sequence coding for the desired protein is inserted under the control of promoters specific for the mammary gland, or through the direct transformation of the mammary glandular epithelium of non-transgenic animals by employing adenovirus vectors.

In other preferred embodiment, chimeric antigens based on HA fused to the extra-cellular domain of the CD154 molecule are produced in the culture of genetically modified yeast, or in mammalian cell culture transduced with an adenovirus vector containing the coding gene.

Also are object of this invention, the vaccine compositions capable of producing a protective immune response against the influenza virus in birds and mammalians, which contain the chimeric antigens previously described. In a particular embodiment of the invention, such vaccine compositions produce a protective immune response against the influenza virus in birds, pigs and humans. The vaccine composition could be administered to animals including man, in a preventive form, by systemic or mucosal route. Through the vaccination with such compositions enormous human, material and economical losses associated with the infection of the influenza virus are avoided.

### Brief description of the drawings

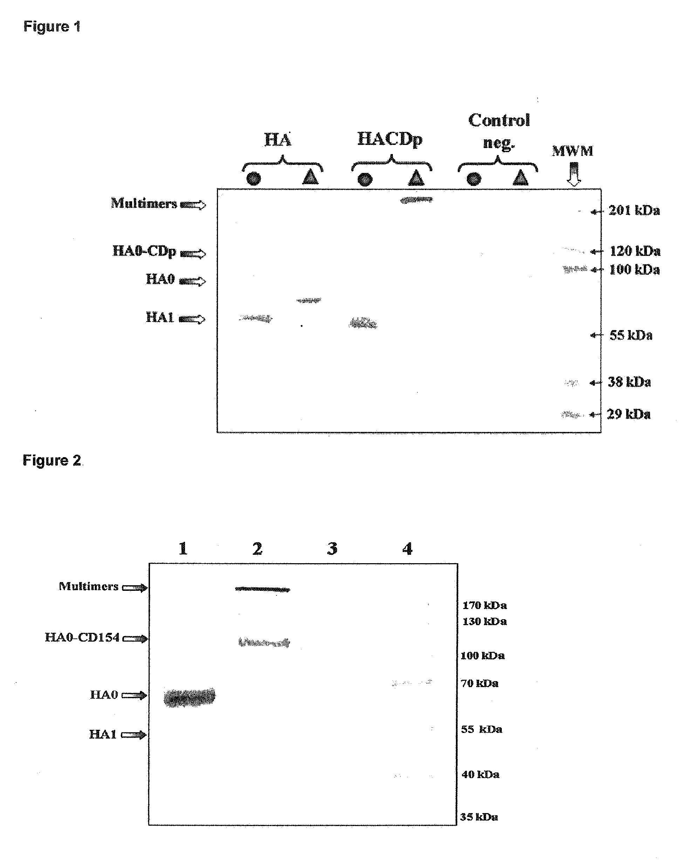

**Figure 1****.** Expression of HA and HACDp antigens in the milk of goats transduced with AdHA and AdHACDp adenoviral vectors, respectively. Proteins present on the milk serum samples of the day 5 post-adenovirus transduction, were separated by SDS-PAGE at 7.5% under reducing (•) or non reducing (Δ) conditions. Immune identification of antigens HA and HACDp was made by "Western-blot" with a hyperimmune chicken serum against a H5N3 strain.
**Figure 2****.** Expression analysis of the recombinant *P. pastoris* clones showing a phenotype Mut^{S}. Western blot of the desired proteins present in the culture media. Lane 1: HA; lane 2: HACDh; lane 3: non transformed MP36; lane 4: Molecular Weight Marker.

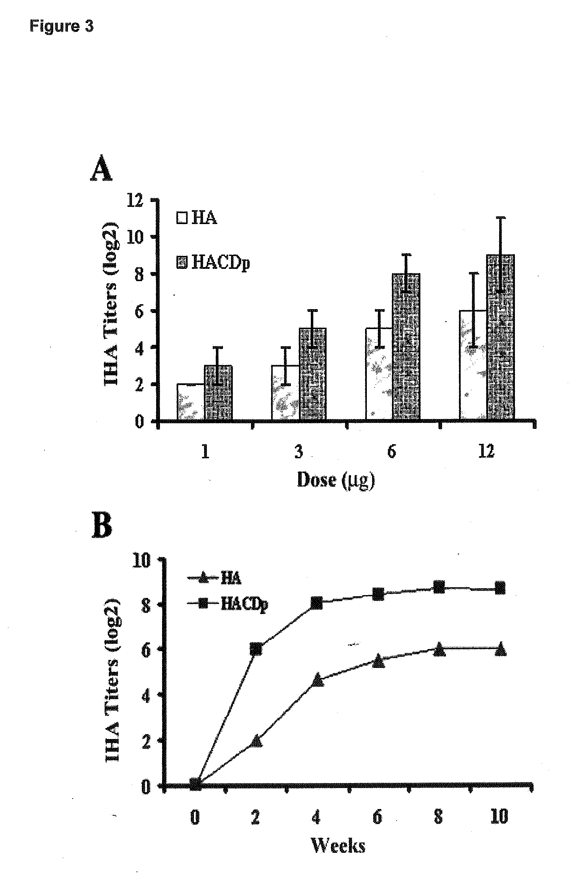

**Figure 3****.** Comparison of the immune response in vaccinated chickens with the variants HA and HACDp. (A) Eight experimental groups of 10 chickens each were taken, the groups received a unique subcutaneous dose of 1, 3, 6 or 12 µg of HA or HACDp. On day 28 post-vaccination, titers of antibodies inhibitors of the hemagglutination were determined. The results are shown as the arithmetical mean +/- the standard deviation of each group. (B) Kinetics of antibodies inhibitors of the hemagglutination in chickens vaccinated with 6 µg of HA and HACDp. Data are shown as an arithmetical mean of all animals from the group on each sampling.

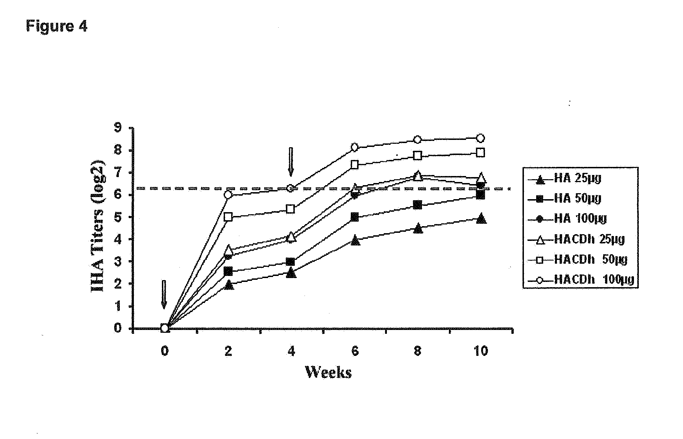

**Figure 4****.** Kinetics of the IHA antibodies. Vaccines were administered on the weeks indicated by arrows. The discontinuous line indicates a titer of 1:80.

### Examples

### Example 1: Obtainment of gene segments encoding for the extracellular domains of hemagglutinin from the virus A/Viet Nam/1203/2004, and human, swine and chicken CD154 molecule.

The coding sequence for the HA molecule derived from the highly pathogenic virus A/Viet Nam 1203/2004 was chemically synthesized. The primary protein sequence was taken from the database of the National Center for Biotechnology Information, (NCBI), access number AY818135. Synthesis was made employing an optimized codon's usage for expression in *Capra hircus*, sequence identified on the Listing Sequences as Seq ID No. 1. The synthetic gene codes for the amino acid sequence of HA from amino acid 1 until 537, eliminating the transmembrane and cytoplasmatic domains of the protein (Seq ID No. 2). During the gene synthesis, additional restriction sites Kpn I and Xho I were incorporated in the 5' extreme of the coding sequence. With the aim of increasing the translation efficiency, a Kozak consensus sequence was incorporated just before the start codon. On extreme 3' of the HA coding fragment were included in the following order: a Nhe I restriction site, the coding fragment for a 6 histidines fragment, a restriction site EcoR I, termination codon, and a restriction site EcoR V.

The coding sequences for the extracellular domain of the human, swine and chicken CD154 molecules were also chemically synthesized. Synthesis was made according to references AJ243435 (for *Gallus gallus*), AB040443 (for *Sus scrofa*) and X67878 (for *Homo sapiens*), from the NCBI database. An optimized codon usage for expression in *Capra hircus* was used for the synthesis of the chicken CD154 (Seq ID. No. 5) and swine CD154 (Seq ID. No. 7) molecules. The codon usage of the human CD154 (Seq ID. No. 3) was not modified. A segment that encodes for a peptide composed by four repeated units of Gly-Gly-Gly-Gly-Ser was included on the amino terminal end of the three molecules with the aim of ensuring their steric freedom.

A restriction site EcoR I was included on the 5' extreme of the coding fragment for extracellular domains of CD154 molecules. A Sal I restriction site was incorporated on 3' extreme (just after the stop codon). The resulting polypeptides derived from synthetic nucleotide sequences appear identified on the Sequences Listing as Seq ID. No. 6 (for *Gallus gallus*), Seq ID. No. 8 (for *Sus scrofa*) and Seq ID. No. 4 (for *Homo sapiens*).

### Example 2: Building of the hemagglutinin expression cassette.

The artificial gene of HA was Kpn I/ EcoR V digested and then inserted in the expression vector pAEC-SPT (Herrera et al. (2000) Biochem Biophys. Res. Commun. 279: 548-551) previously digested with the same enzymes. The resulting vector was denominated pHA. Vector pHA was digested with restriction endonuclease EcoR I (that cuts after the histidines tail just before the termination codon of HA) and with the endonuclease Sal I (that cuts on the multiple cloning site of vector pHA to the 3' of HA). CD154 genes were removed from the plasmid vectors supplied by GeneArt with the endonucleases Eco RI and Sal I and were cloned on the vector pHA. From these cloning were generated three mammalian cells expression vectors: pHA-CDp (contains the domain of chicken CD154), pHA-CDc (contains the domain of swine CD154), pHA-CDh (contains the domain of human CD154). In all cases chimeric genes of HA were under the control of the Cytomegalovirus Immediate-Early Promoter (pCMV).

### Example 3: Building of adenoviral vectors (ΔE1ΔE3) containing the HA gene.

Replication defective adenoviral vectors were built based on the AdEasy system (Tong-Chuan H et al. (1998). A simplified system for generating recombinant adenoviruses PNAS USA, 95: 2509-2514). The plasmid pAdTrack-CMV was employed as a transfer vector. The system based on AdEasy constitutes a rapid and simple alternative of recombinant adenovirus construction. Coding sequences for HA and for HA fused to the extracellular domains of human, swine and chicken variants of the CD154 molecule were removed with the endonucleases Xho I and Sal I and cloned on the Echo I site from pAdTrack-CMV vector. Resulting vectors (ptrack-HA, ptrack-HACDp, ptrack-HACDh, ptrack-HACDc) were linealized by Pme I digestion and co-electropored with the pAdEasy vector into the bacterial strain BJ5183. In order to obtain infective virions, the recombinant viral genomes were digested with the endonuclease Pac I and transfected on the HEK-293 cell line. Four virus vectors: Ad-HA, Ad-HACDp, Ad-HACDh and AdHACDc were generated. All vectors were amplified in the HEK-293 cell line until a titer of 5x10¹² colony forming units (CFU) was reach. The produced virus was purified by double centrifugation in CsCl, was dialyzed against storage buffer (10 mM Tris pH 8.0, 2 mM MgCl₂, 4% Sacarose) and was kept at -70°C for its subsequent usage.

### Example 4: Direct Transduction of the Goat Mammary Gland Epithelium:

Goats used were on the third month lactation and producing an average of 1.3 liters of milk daily. On day 0, animals received a dose of 10 mg of diazepam, by intramuscular route, to decrease the stress during treatment. Animals were extensively milked in order to eliminate most of milk on cisterns; mammary glands were rinsed twice by infusion with saline solution at 37°C and subsequent milking. All infusions were done directly through the nipple's channel using a catheter coupled to a peristaltic pump. Infusions were made slowly while in a simultaneous way massages were applied on the infused udders.

On goats, udder can be separated in two independent halves. A solution of PBS supplements with 30 mM of EGTA and containing a virus load of 10⁹ CFU/ml was infused into each mammary gland. The volume infused on each udder's half was variable depending of the udder capacity (as average, 600 ml by udder's medium). After the infusion, massages were applied to udder to facilitate that the solution were homogenously distributed reaching the totality of ducts and alveolus. In the next day the solution infused was removed by milking. Mammary glands were rinsed again by PBS infusion with the purpose of eliminating the greatest amount of adenovirus vectors remaining on the cistern and mammary ducts.

The collection of milk from infused animals began 48 hours post infusion and was performed by manual milking. Two milking were made daily, one in the morning and the other at the end of the afternoon. Most of the collected milk was stored at -70°C for the subsequent protein purification, while small samples were used to detect and quantify the content of HA variants on each batch.

Detection of HA variants on milk was made as follows. Four volumes of separation buffer (10 mM Tris-HCl, 10mM CaCl₂) were added to 150 µl samples of milk, after incubation during 30 minutes on ice, samples were centrifuged at 4°C during 30 minutes at 15 000g. The serum fraction was recovered and 10 µl of the proteins were separated on a 7% Sodium Dodecyl Sulphate-Polyacrylamide Gel Electrophoresis. (SDS-PAGE). Proteins were transferred to a nitrocellulose filter and the presence of HA was detected by the employing chicken hyperimmune serum. As a secondary antibody was used a mouse anti-chicken antibody conjugated to Horseradish Peroxidase (HRP). Immunoreactive bands were visualized by Enhanced Chemiluminiscence (ECL) from Amersham Pharmacia Biotech (Fig. 1). Variants of HA were produced mostly in polypeptide chains (HA0 and HA0-CD154) form, although it can be observed also the presence of the domain HA1 in both molecules.

Quantification of HA variants was made by an H5-specific ELISA developed at CIGB. In animals infused with a E1ΔE3 adenoviral vectors, variants of HA were detected until day 11 post infusion, with an expression average of 0.94 g/L, 0.86 g/L, 0.78 g/L, 0.87 g/L for the HA, HACDp, HACDh, and HACDc proteins respectively, during the first 7 days of collection.

### Example 5: Constriction of Pichia pastoris expression vectors.

The *P. pastoris* expression vector pPS10 was enzymatically digested with the restriction endonuclease Nae I, and then treated with alkaline phosphate for end dephosphorylation and further insertion of HA, HACDp, HACDh, and HACDc coding genes.

Coding sequences for proteins HA, HACDp, HACDh, and HACDc, were removed by digestion with the endonuclease Bcl I (which cut site is found immediately after the secretion peptide of HA) and Sma I (which cut site is found just after the stop codon). Resulting ends were treated with Klenow polymerase in order to blunt the extremes, and then each band was cloned in the pPS10 vector, obtaining the pPS-HA, pPS-HACDp, pPS-HACDh and pPS-HACDc yeast expression vectors. In all cases, coding sequences for the different variants of HA were under the control of an alcohol oxidase promoter (AOX1) and fused to a secretion peptide from Suc2 of *Saccharomyces cerevisiae*.

Before transformation, plasmids were opened by digestion with the restriction endonuclease Sph I. Strain MP36 of *P. pastoris* was transformed by electrophoration with the expression vectors pPS-HA, pPS-HACDp, pPS-HACDh and pPS-HACDc. Such strain is an auxotrophic mutant *his3,* which after transformation acquires a phenotype His+.

Transformed clones identified by Dot Blot were analyzed by Southern Blot to determine in which had occurred the integration by the replacement of gene AOX1 of *P. pastoris* for the expression cassette of recombinant plasmid, which is in correspondence with a phenotype Mut^{S} (low usage of methanol) and His+. Gene replacement of AOX1 occurs by crossing over of the promoters regions of AOX1 and 3'AOX1 between vector and genome.

Because of these crossings over, the deletion of the coding region of gene AOX1 occurs. Recombinant strains with phenotype Mut^{S} fix the production of oxidase alcohol (AOX) on gene AOX2 and its growing rate on methanol is low.

Genes that encode for the different variants of HA are under the regulation of the promoter AOX1, which is inducible through methanol. *P. pastoris* secretes only low levels of own proteins and its culture medium does not need protein supplements, so then, it can be expected that an heterologous protein that is secreted, constitutes the majority of the total proteins in the culture medium (until the 80%). Production of recombinant antigens was made on 5L fermentors by addition of methanol to the culture and keeping culture's pH in 8.0. As it is shown in Figure 2, the greater part of HA produced in *P. pastoris* was glycosilated, and secreted to the culture medium.

### Example 6: Purification of HA, HACDp, HACDH, and HACDc antigens.

For the purification of HA, HACDh, HACDh and HACDc from goat milk, the fat was removed by centrifugation at 10 000 rpm, at 4°C during 30 minutes. Fat free milk was diluted 1:5 (v/v) in buffer Tris-HCl pH 8.0, CaCl₂ 10 mM. After one hour at 4°C, the caseins were precipitated by centrifugation at 10 000 during 20 minutes.

Milk sera containing each from the antigens were clarified by serial filtration on glass prefilters, filters of 5µM, 0.8µM and 2µM. Clarified serums were diafiltrated against a phosphate buffer (50 mM NaH₂PO₄ pH 8.0, 150 nM NaCl, 10mM imidazol) and were loaded on a Ni-NTA Sepharose column. A washing step with 50 mM of imidazol was made and recombinant proteins were eluted at 200mM of imidazol on phosphate buffer.

For purification of antigens HA, HACDp, HACDh and HACDc, produced on *P. pastoris*, once concluded the fermentation, cells were separated from the culture medium through centrifugation. Media containing recombinant antigens were clarified by serial filtration in filters of 5µM, 0.8µM and 2µM. The rest of the purification was made in a way similar to the proceeding followed to purify the antigens from the milk.

### Example 7: Assessment of the immunogenicity in chickens of vaccine compositions based on the HA and HACD vaccine antigens.

Immunization: HA and HACDp antigens used on this trial were purified by non-denaturalizing methods until more than 98% of purity, as it could be determined by densitometry analysis of a SDS-PAGE. Identity of proteins was confirmed by the amino acid analysis through mass spectrometry, and by "Western blot" using a hyperimmune serum against H5. Both antigens were formulated in the oil adjuvant Montanide ISA 720. For immunization, chickens of three weeks of age were used. According to the dose used, 8 groups of 10 chickens each were created. Four of the groups received the formulation based on antigen HA, while the others four received the formulation based on HACDp. Animals were immunized by the administration of 1µg, 3 µg, 6 µg, or 12 µg of antigen through subcutaneous route, according to the group, with 200 µl of the final formulation. A needle 18G coupled to a syringe of 1ml was used for immunization. To this trial was incorporated a placebo group that does not receive the antigen. Blood samples for the analysis of serological response were taken on day 28 after vaccination.

Hemagglutination Inhibition and ELISA: For inhibition of hemagglutination assay (IHA), serums were serially diluted (initial dilution 1:2) in U-bottom microtitre plates. To each well were added 4 hemagglutinin units of inactivated virus antigen A/Viet Nam/1203/2004 (previously determined by titration against a suspension of chicken erythrocytes at 0.5% in Phosphate Buffer Saline (PBS)). The mixture was incubated at room temperature during 1 hour and once this time passed, a similar volume of chickens' erythrocytes at 0.5% in PBS were added to each well. Titers inhibitors of hemagglutination were read 30 minutes later. Antibodies specific against H5 were determined by ELISA. Plates were coated with 0,5 µg /well of HA protein produced and purified from the cell cultures infected with AdHA vector. Titers obtained by ELISA were expressed as the higher dilution that rendered an optical density superior to the double of the mean plus the standard deviation, from negative samples diluted in similar way.

Cytokines Expression assay: The spleens from chickens immunized with 6µg of HA or HACDp were aseptic collected on day 30 post-vaccination. In order to obtain an homogeneous cellular suspension, spleens were cut into little fragments and were passed through a steel filter with a porous size of 120 µM. Cells were collected by centrifugation at 1000 during 10 minutes and suspended in PBS. The cell suspension was applied slowly over an Histopaque column 1083 (Sigma) in a relation 1:1 (v/v) and then centrifuged at 1000 rpm for 30 minutes. Mononuclear cells were collected on the interphase ring, were washed with PBS three times and were adjusted to a concentration of 1x 10⁷ cells per milliliter of RPMI 1640 medium. Cells were seeded on 24 well plates at the rate of 5x10⁶ cells per well.

To perform the lymphoproliferation assay, cells were stimulated by addition of protein HA at a concentration of 1µg/ml, during 18 hours at 41°C on 5% of CO₂. As negative control, cells from spleen of chicken vaccinated with placebo were taken. As positive control, spleen cells incubated with Concanavaline A (Con A) were used. Cultures were collected 18 hours later and the total ribonucleic acid (RNA) purified to evaluate the induction of cytokine genes. Total RNA was purified by using the Tri-Reagent (Sigma) method. Samples of RNA were suspended in water and quantified by spectrometry at 260 nm.

With the aim of determining the relative levels of RNA messenger of interleukin 2 (IL-2), Interferon Gamma (IFN-γ) and glyceraldehyde-phosphodehydrogenase (GAPDH), a reverse transcription assay and further PCR (RT-PCR) was made on triplicate, as described Svetic and co-workers in 1991, (Svetic, A. et al. (1991), Cytokine Gene Expression after in vivo primary immunization with goat antibody to mouse IgD antibody. J. Immunol. 147:2391-2397). RNA was retrotranscripted by triplicate using a Reverse Transcription System kit (Promega), according to the manufacturer's specifications. In a previous study a profile of the amount of DNA amplified in each cycle of PCR, for every gene to be analyzed, was made. The cycle's number used was selected inside the exponential region of the amplification curve. The optimal number of cycles was 35 for IL-12 and IFN-γ, while for GAPDH was 28.

Both, the positive control cells (incubated with Con A) and negative control cells (derived from a placebo group and un-stimulated) were included on each trial. Constitutive gene GAPDH was amplified on each PCR and used to guarantee similar amounts of initial complementary ADN on every reaction before evaluating genes of interest.

After PCR, 15 µl of the final reaction was analyzed by 2% Agarose Gel Electrophoresis and semiquantified by densitometry analysis. Intensity of the bands was determined using the computational program of image analysis "Kodak 1 D". Results were reported as mean of the intensity values of pixels in the interest band, and as the relative expression of gene estimated as the average of this value with respect to the intensity obtained for the constitutive gene GAPDH.

Results obtained in chickens indicate that the water in oil formulation containing vaccine antigens HA and HACDp induced a humoral and cellular immune response on vaccinated animals. In both cases, the immune response was dependent of the dose employed (Fig. 3A). Kinetics of antibodies inhibitors of hemagglutination in animals vaccinated with 6µg of HA or HACDp showed that in week 4 post-vaccination, the chimeric antigen HACDp is able to induce a response of IHA antibodies about 10 times higher to the one developed in animals vaccinated with the HA antigen (Fig. 3B). Also, on animals vaccinated with the chimeric antigen HACDp was observed an expression highly marked of IFN-γ and IL-12, when mononuclear cells of peripheral blood were exposed to antigen HA (Table 1). This result shows that the fusion of CD154 to HA is capable of inducing a strong response at the cellular level, specific against the HA molecule.

**Table 1: Cell-mediated immune response.**

| Dose (µg) | GAPDH | IFN-γ | | | IL-12 | | |
|---|---|---|---|---|---|---|---|
| | | HA | HACDp | Relation HACD/HA | HA | HACDp | Relation HACD/HA |
| 1 | 19850 | 3156 | 17015 | 5,39 | 2780 | 9232 | 3,32 |
| 3 | 22735 | 3828 | 19210 | 5,02 | 2505 | 11420 | 4,55 |
| 6 | 20173 | 5985 | 26525 | 4,43 | 3423 | 13395 | 3,91 |
| 12 | 21871 | 6215 | 28905 | 4,65 | 3928 | 16683 | 4,24 |

The relative levels of IFN-γ and IL-12 *in vitro* production are shown as the arithmetic mean of all the animals in the group.

### Example 8: Immunogenicity in humans of vaccine compositions based on HA and HACDh antigens.

Safety, reactogenicity, and immunogenicity of vaccine compositions that contain HA and HACDh proteins were evaluated on clinical trials in humans. Both proteins were obtained with a purity level higher than 98.5% and were formulated absorbed to aluminum hydroxide. As a selection criterion, healthy male persons, among 25 and 40 years old, which have not received any vaccination in the last 6 months and do not show any influenza symptom were employed. Six experimental groups of 8 persons each were created on the basis of dose (25 µg, 50 µg, or 100 µg) and the type of antigen to be received (HA or HACDh). On week 0 every volunteer was immunized and 4 weeks later they received a second immunization. Administration was made by intramuscular injection of 0.5 ml of composition.

Blood samples were taken at the time of vaccination and during the three following weeks, with two weeks intervals. For determination of levels of the antibodies inhibitors of hemagglutination, serum samples were treated with receptor-destroying enzyme from *Vibrio cholera,* and subsequently heated at 65°C for the inactivation of non-specific inhibitors. Antibodies inhibiting hemagglutination for the virus A antigen/Viet Nam/1203/2004 were determined by the standard assay of microtitration using chicken erythrocytes at 0.5%.

IgG immunoglobulin levels specific against H5 protein from virus ANiet Nam/1203/2004 were evaluated by ELISA. Plates were coated with HA protein produced in cell cultures. Next, serial dilutions from each serum were made. After washing extensively, an anti-human IgG antibody produced in rabbits and conjugated with HRP was added to each well. Development was made with 3,3',5,5' Tetramethyl Benzedine. ELISA titers ware expressed as the higher dilution to which the optical density of the wells containing antigen was at least the double of corresponding well without antigen.

Peripheral Blood Mononuclear Cells (PBMC) were isolated by separation on a Ficoll Isopaque gradient (ICN Biomedical Inc. Aurora OH) and were cryopreserved for immunological assays. After thawing, PBMC were used for a INF-γ ELISPOT (Ebioscience) according to the manufacture's instructions. Briefly, plates were coated overnight with the capture antibody. After two washing steps, plates were blocked with RPMI-1640 medium during an hour. In every well HA antigen was added and 5x10⁵ cells were seeded. Culture was incubated at 37°C, 5% CO₂ during 48 hours. The cells were decantanted and plates were incubated with an anti-interferon antibody conjugated to biotin for 2 hours. After two washing steps, avidin conjugated to HRP was added to each well. Development was made using a solution containing 3-amino-9-ethyl carbazole as substrate. Results showed that vaccines containing HA and HACDh recombinant antigens did not produce local adverse reactions.

In volunteers immunized with HA as in those immunized with HACDh chimeric protein, the cellular and humoral immune response increased proportionally with the dose of protein administrated. Nevertheless, comparison between HA and HACDh variants showed, that chimeric protein is capable of inducing a humoral response 4.2 times higher to the equivalent dose of HA (Fig. 4), an a cellular response 5.2 times higher than the response produce by the equivalent dose of HA.

### Example 9: Immunogenicity in Swine of vaccine compositions based on HA and HACDc antigens.

Landrace pigs weighing between 18-20 kg were used. Animals were distributed in six experimental groups, according to the antigen and the dose to be evaluated. Eight pigs were employed per experimental group. For HA and HACDc, doses of 20µg, 40 µg and 80 µg were evaluated. Both vaccine antigens were formulated as a water in oil emulsion and inoculated by injection in the neck muscle with 2-ml doses. The Placebo composed by adjuvant and phosphate saline solution 1:1 (v/v), was inoculated in a similar way.

Blood samples were taken at the moment of vaccination, and every 7 days during the next three months. Before determining the antibody titers in IHA, serum samples were treated with receptor-destroying enzyme from *Vibrio cholera* in order to inactivate non-specific inhibitors. Antibody titers in IHA were determined by the standard assay of microtitration using chicken erythrocytes at 0.5%. Immune response at the cell level was evaluated through the relative estimation of RNA levels of IFN-γ and IL-12, in PBMC stimulated with the HA antigen.

Non alteration of the normal clinical parameters was observed in the immunized animals which suggests that there is no adverse response to the vaccine composition. Results showed that vaccines containing recombinant antigens HA and HACDc produced a very little adverse reactions, of local character. In both groups, was observed a dependence of the dose with IHA antibody titers and the cell response expressed as the capacity of the cells to proliferate and produce cytokines in presence of HA antigen. In the groups immunized with HACDc chimeric antigen, a remarkable potentiation of the immune response was observed, not only at humoral but also at cellular level. Such protein was capable of inducing titers of antibodies in IHA 2.5 times higher than those developed by the correspondent dose of HA. The cell response capacity, relative to the IFN-γ and IL-2 expression levels also was of 4.5 and 6 times higher, respectively, in animals immunized with HACDc chimeric antigen, in comparison to those in pigs immunized with an equivalent dose of HA.

### Example 10: Expression of chimeric antigens based on Hemagglutinin from different subtypes of Avian Influenza Virus.

Coding nucleotide sequences were synthesized for HA extracellular domains from avian influenza viruses A/Netherlands/33/03(H7N7) (Seq ID. No. 9) and A/Hong Kong/1073/99 (H9N2) (Seq ID. No. 10).

Both genes were designed with a codon's usage optimal for expression in *Capra hircus,* and flanked by restriction sites Xho I (in 5') and EcoR I (in 3'). Both genes were directly cloned on adenovirus transfer vector pAdtrack containing the extracellular domain of swine CD154 molecule. The resulting recombinant clones were transfected on the HEK-293T cell line and 72 hours later the expression of chimeric molecules could be detected in the culture medium. Both fusion proteins were mainly expressed as trimers and could be purified from the culture medium by a single step of Immobilized metal ion affinity chromatography.

Adenovirus transfer vectors containing chimeric variants of HA from viruses A/Netherlands/33/03 (H7N7) and Hong Kong/1073/99 (H9N2) were integrated by homologous recombination in the adenoviral genome contained on pAdEasy plasmid vector from which were generated and amplified adenovirus vectors containing both proteins.

HA from the isolates mentioned before, fused to the extracellular domain of CD154, were produced at higher concentrations in goat milk. This strategy allowed to produce several grams of chimeric variants of HA in a term shorter than 55 days from the moment in which the synthetic genes were avalilable.

After the purification of both chimeric antigens, an immunogenicity experiment in pigs was made using a fixed dose of 20 µg per animal, in a water in oil formulation administered by intramuscular injection in the neck muscle with 2-ml doses. Adverse reactions were not observed. Serological analysis demonstrated that both proteins were capable of inducing a strong humoral immune response, reaching IHA antibodies titers higher than 1:800 within the first 28 days post-vaccination.

Sequence Listing

<110> CENTRO DE INGENIERIA GENETICA Y BIOTECNOLOGIA

<120> CHIMERIC VACCINE ANTIGENS AGAINST AVIAN INFLUENZA VIRUS.

<130> P85766EP00

<150> PCT/CU2007/000009
<151> 2007-02-28

<150> CU 2006-0051
<151> 2006-02-28

<160> 10

<170> PatentIn Ver. 2.1

<210> 1
<211> 1670
<212> ADN
<213> Artificial Sequence

<220>
<223> Artificial sequence description: Synthetic sequence
      which codifies for the hemagglutinin of the
      Viet Nam/1203/2004 virus fused to a 6 histidines
      tail and flanked by several restriction sites.

<400> 1
ggtaccctcg agccaccatg gagaagatcg tgctgctgtt cgccatcgtg tccctggtga 60
agagtgatca gatctgtatc ggctaccacg ccaacaactc caccgagcag gtggacacca 120
tcatggaaaa gaacgtgacc gtgacccacg cccaggacat cctggagaag aagcacaacg 180
gcaagctgtg tgacctggac ggcgtgaagc ccctgatcct gagggactgc tccgtggccg 240
gctggctgct gggcaacccc atgtgtgacg agttcatcaa cgtgcccgag tggtcctaca 300
tcgtggagaa ggccaacccc gtgaacgacc tgtgctaccc cggcgacttc aacgactacg 360
aggagctgaa gcacctgctg tccaggatca accacttcga gaagatccag atcatcccca 420
agtccagctg gtcctcccac gaggcctccc tgggcgtgtc ctccgcctgc ccctaccagg 480
gcaagtcctc cttcttcaga aacgttgtgt ggctgattaa gaagaacagc acctacccca 540
ccatcaagag gtcctacaac aacaccaacc aggaggacct gctggttctg tggggcatcc 600
accaccccaa cgacgccgcc gagcagacca agctgtacca gaaccccacc acctacatct 660
ctgtgggcac ctccaccctg aaccagaggc tggtgcccag gatcgccacg cgttccaagg 720
tgaacggcca gagcggccga atggagtttt tctggaccat cctgaagcca aacgacgcca 780
tcaacttcga gtccaacggc aacttcatcg cccccgagta cgcctacaag atcgtgaaga 840
agggcgactc caccatcatg aagtccgagc tggagtacgg caactgtaac accaagtgcc 900
agaccccaat gggcgccatc aacagctcca tgcccttcca caacatccac cccctgacca 960
tcggcgagtg ccccaagtac gtgaagtcca acaggctggt gctggccacc ggcctgagga 1020
actcccccca gagggagagg aggaggaaga agaggggcct gttcggcgcc atcgccggct 1080
tcatcgaggg cggctggcag ggcatggtgg acggctggta cggctaccac cacagcaacg 1140
agcagggctc cggctacgcc gccgacaagg agtccaccca gaaggccatc gacggcgtca 1200
ccaacaaggt gaactccatc atcgacaaga tgaacaccca gttcgaggct gtgggcaggg 1260
agttcaacaa cctggagagg agaatcgaga acctgaacaa gaagatggag gacggcttcc 1320
tggatgtgtg gacctacaac gccgagctgc tggtgctgat ggagaacgag aggaccctgg 1380
acttccacga ctccaacgtg aagaacctgt acgacaaggt gaggctgcag ctgagggaca 1440
acgccaagga gctgggcaac ggctgcttcg agttctacca caagtgtgac aacgagtgta 1500
tggagtctgt gaggaacggc acctacgact acccccagta ctccgaggag gccaggctga 1560
agagggagga gatctctggc gtgaagctgg agtccatcgg catctaccag atcctgtcca 1620
tctactccgc tagccatcac catcaccacc atatgaattc ttgagatatc 1670

<210> 2
<211> 548
<212> PRT
<213> Influenza A virus

<220>
<221> PEPTIDE
<222> (1)..(548)

<223> Amino acid sequence (starting by the start codon
      "ATG" [nucleotide 18] of the Seq ID. No
      1).

<400> 2
Met Glu Lys Ile Val Leu Leu Phe Ala Ile Val Ser Leu Val Lys Ser
1               5                   10                  15

Asp Gln Ile Cys Ile Gly Tyr His Ala Asn Asn Ser Thr Glu Gln Val
            20                  25                  30

Asp Thr Ile Met Glu Lys Asn Val Thr Val Thr His Ala Gln Asp Ile
        35                  40                  45

Leu Glu Lys Lys His Asn Gly Lys Leu Cys Asp Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Asn Pro Val Asn Asp Leu Cys Tyr Pro Gly Asp Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Ser Ser His Glu Ala Ser
    130                 135                 140

Leu Gly Val Ser Ser Ala Cys Pro Tyr Gln Gly Lys Ser Ser Phe Phe
145                 150                 155                 160

Arg Asn Val Val Trp Leu Ile Lys Lys Asn Ser Thr Tyr Pro Thr Ile
                165                 170                 175

Lys Arg Ser Tyr Asn Asn Thr Asn Gln Glu Asp Leu Leu Val Leu Trp
            180                 185                 190

Gly Ile His His Pro Asn Asp Ala Ala Glu Gln Thr Lys Leu Tyr Gln
        195                 200                 205

Asn Pro Thr Thr Tyr Ile Ser Val Gly Thr Ser Thr Leu Asn Gln Arg
    210                 215                 220

Leu Val Pro Arg Ile Ala Thr Arg Ser Lys Val Asn Gly Gln Ser Gly
225                 230                 235                 240

Arg Met Glu Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Thr Ile Met Lys Ser Glu Leu Glu Tyr Gly
        275                 280                 285

Asn Cys Asn Thr Lys Cys Gln Thr Pro Met Gly Ala Ile Asn Ser Ser
    290                 295                 300

Met Pro Phe His Asn Ile His Pro Leu Thr Ile Gly Glu Cys Pro Lys
305                 310                 315                 320

Tyr Val Lys Ser Asn Arg Leu Val Leu Ala Thr Gly Leu Arg Asn Ser
                325                 330                 335

Pro Gln Arg Glu Arg Arg Arg Lys Lys Arg Gly Leu Phe Gly Ala Ile
            340                 345                 350

Ala Gly Phe Ile Glu Gly Gly Trp Gln Gly Met Val Asp Gly Trp Tyr
        355                 360                 365

Gly Tyr His His Ser Asn Glu Gln Gly Ser Gly Tyr Ala Ala Asp Lys
    370                 375                 380

Glu Ser Thr Gln Lys Ala Ile Asp Gly Val Thr Asn Lys Val Asn Ser
385                 390                 395                 400

Ile Ile Asp Lys Met Asn Thr Gln Phe Glu Ala Val Gly Arg Glu Phe
                405                 410                 415

Asn Asn Leu Glu Arg Arg Ile Glu Asn Leu Asn Lys Lys Met Glu Asp
            420                 425                 430

Gly Phe Leu Asp Val Trp Thr Tyr Asn Ala Glu Leu Leu Val Leu Met
        435                 440                 445

Glu Asn Glu Arg Thr Leu Asp Phe His Asp Ser Asn Val Lys Asn Leu
    450                 455                 460

Tyr Asp Lys Val Arg Leu Gln Leu Arg Asp Asn Ala Lys Glu Leu Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Gln Tyr Ser Glu Glu Ala
            500                 505                 510

Arg Leu Lys Arg Glu Glu Ile Ser Gly Val Lys Leu Glu Ser Ile Gly
        515                 520                 525

Ile Tyr Gln Ile Leu Ser Ile Tyr Ser Ala Ser His His His His His
    530                 535                 540

His Met Asn Ser
545


<210> 3
<211> 693
<212> DNA
<213> Artificial sequence

<220>
<223> Artificial Sequence Description: Synthetic
      sequence that codicies for the extracellular
      domain of the CD154 molecule of Homo sapiens
      containing a spacer peptide in its N-terminal end.S

<400> 3
gaattctggc ggcggctccg gagggggagg gagcggcgga gggggctcca agatagaaga 60
tgaaaggaat cttcatgaag attttgtatt catgaaaacg atacagagat gcaacacagg 120
agaaagatcc ttatccttac tgaactgtga ggagattaaa agccagtttg aaggctttgt 180
gaaggatata atgttaaaca aagaggagac gaagaaagaa aacagctttg aaatgcaaaa 240
aggtgatcag aatcctcaaa ttgcggcaca tgtcataagt gaggccagca gtaaaacaac 300
atctgtgtta cagtgggctg aaaaaggata ctacaccatg agcaacaact tggtaaccct 360
ggaaaatggg aaacagctga ccgttaaaag acaaggactc tattatatct atgcccaagt 420
caccttctgt tccaatcggg aagcttcgag tcaagctcca tttatagcca gcctctgcct 480
aaagtccccc ggtagattcg agagaatctt actcagagct gcaaataccc acagttccgc 540
caaaccttgc gggcaacaat ccattcactt gggaggagta tttgaattgc aaccaggtgc 600
ttcggtgttt gtcaatgtga ctgatccaag ccaagtgagc catggcactg gcttcacgtc 660
ctttggctta ctcaaactct gacccgggtc gac                              693

<210> 4
<211> 225
<212> PRT
<213> Homo sapiens

<220>
<221> PEPTIDE
<222> (1)..(225)
<223> Amino acid sequence (starting by the codon
      "TCT" [nucleotide 5] of the Seq ID. No 3).

<400> 4
Ser Gly Gly Gly Ser Gly Gly Gly Gly Ser Gly Gly Gly Gly Ser Lys
1               5                   10                  15

Ile Glu Asp Glu Arg Asn Leu His Glu Asp Phe Val Phe Met Lys Thr
            20                  25                  30

Ile Gln Arg Cys Asn Thr Gly Glu Arg Ser Leu Ser Leu Leu Asn Cys
        35                  40                  45

Glu Glu Ile Lys Ser Gln Phe Glu Gly Phe Val Lys Asp Ile Met Leu
    50                  55                  60

Asn Lys Glu Glu Thr Lys Lys Glu Asn Ser Phe Glu Met Gln Lys Gly
65                  70                  75                  80

Asp Gln Asn Pro Gln Ile Ala Ala His Val Ile Ser Glu Ala Ser Ser
                85                  90                  95

Lys Thr Thr Ser Val Leu Gln Trp Ala Glu Lys Gly Tyr Tyr Thr Met
            100                 105                 110

Ser Asn Asn Leu Val Thr Leu Glu Asn Gly Lys Gln Leu Thr Val Lys
        115                 120                 125

Arg Gln Gly Leu Tyr Tyr Ile Tyr Ala Gln Val Thr Phe Cys Ser Asn
    130                 135                 140

Arg Glu Ala Ser Ser Gln Ala Pro Phe Ile Ala Ser Leu Cys Leu Lys
145                 150                 155                 160

Ser Pro Gly Arg Phe Glu Arg Ile Leu Leu Arg Ala Ala Asn Thr His
                165                 170                 175

Ser Ser Ala Lys Pro Cys Gly Gln Gln Ser Ile His Leu Gly Gly Val
            180                 185                 190

Phe Glu Leu Gln Pro Gly Ala Ser Val Phe Val Asn Val Thr Asp Pro
        195                 200                 205

Ser Gln Val Ser His Gly Thr Gly Phe Thr Ser Phe Gly Leu Leu Lys
    210                 215                 220

Leu
225


<210> 5
<211> 726
<212> DNA
<213> Artificial sequence

<220>
<223> Artificial Sequence Description: Synthetic
      Sequence that codifies for the extracelular domain
      of the CD154 molecule of Gallus gallus containing

an spacer peptide in its N-terminal end.

<400> 5
gaattctggc ggcggctccg gagggggagg gagcggcgga gggggctcca agatggagga 60
ggtgctgtcc ctgaacgagg actacatctt cctgaggaag gtgcagaagt gccagaccgg 120
cgaggaccag aagagcaccc tgctggactg tgagaaggtg ctcaagggct tccaggacct 180
gcagtgcaag gacaggaccg ccagcgagga gctgcccaag ttcgagatgc acaggggcca 240
cgagcacccc cacctgaaga gcaggaacga gaccagcgtc gccgaggaga agaggcagcc 300
catcgccacc cacctggccg gcgtgaagag caacaccact gtgagggtgc tgaagtggat 360
gaccacctcc tacgccccca cctccagctt catcagctac cacgagggca agctgaaggt 420
ggagaaggcc ggcctgtact acatctacag ccaggtgtcc ttctgtacca aggccgctgc 480
cagcgccccc ttcaccctgt acatctacct gtacctgccc atggaggagg acaggctgct 540
gatgaagggc ctggacaccc acagcaccag caccgccctg tgtgagctgc agagcatcag 600
ggagggcggc gtgttcgagc tgaggcaggg cgacatggtg ttcgtgaacg tgaccgacag 660
caccgccgtg aacgtgaacc ccggcaacac ctacttcggc atgttcaagc tgtgacccgg 720
gtcgac 726

<210> 6
<211> 236
<212> PRT
<213> Gallus gallus

<220>
<221> PEPTIDE
<222> (1)..(236)
<223> Amino acid sequence(starting by the
"TCT" codon [nucleotide 5] of the Seq ID. No 5).

<400> 6
Ser Gly Gly Gly Ser Gly Gly Gly Gly Ser Gly Gly Gly Gly Ser Lys
1               5                   10                  15

Met Glu Glu Val Leu Ser Leu Asn Glu Asp Tyr Ile Phe Leu Arg Lys
            20                  25                  30

Val Gln Lys Cys Gln Thr Gly Glu Asp Gln Lys Ser Thr Leu Leu Asp
        35                  40                  45

Cys Glu Lys Val Leu Lys Gly Phe Gln Asp Leu Gln Cys Lys Asp Arg
    50                  55                  60

Thr Ala Ser Glu Glu Leu Pro Lys Phe Glu Met His Arg Gly His Glu
65                  70                  75                  80

His Pro His Leu Lys Ser Arg Asn Glu Thr Ser Val Ala Glu Glu Lys
                85                  90                  95

Arg Gln Pro Ile Ala Thr His Leu Ala Gly Val Lys Ser Asn Thr Thr
            100                 105                 110

Val Arg Val Leu Lys Trp Met Thr Thr Ser Tyr Ala Pro Thr Ser Ser
        115                 120                 125

Phe Ile Ser Tyr His Glu Gly Lys Leu Lys Val Glu Lys Ala Gly Leu
    130                 135                 140

Tyr Tyr Ile Tyr Ser Gln Val Ser Phe Cys Thr Lys Ala Ala Ala Ser
145                 150                 155                 160

Ala Pro Phe Thr Leu Tyr Ile Tyr Leu Tyr Leu Pro Met Glu Glu Asp
                165                 170                 175

Arg Leu Leu Met Lys Gly Leu Asp Thr His Ser Thr Ser Thr Ala Leu
            180                 185                 190

Cys Glu Leu Gln Ser Ile Arg Glu Gly Gly Val Phe Glu Leu Arg Gln
        195                 200                 205

Gly Asp Met Val Phe Val Asn Val Thr Asp Ser Thr Ala Val Asn Val
    210                 215                 220

Asn Pro Gly Asn Thr Tyr Phe Gly Met Phe Lys Leu
225                 230                 235

<210> 7
<211> 693
<212> DNA
<213> Artificial Sequence

<220>
<223> Artificial Sequence Description: Synthetic
      Sequence that codifies for the extracelular domain
      of the CD154 of Sus scrofa  containing an spacer
      peptide in its N-terminal end.

<400> 7
gaattctggc ggcggctccg gagggggagg gagcggcgga gggggctcca agatcgagga 60
cgagaggaac ctgcacgagg acttcgtgtt catcaagacc atccagaggt gtaagcaggg 120
cgagggcagc ctgagcctcc tgaactgtga ggagatcagg agccagttcg aggacctggt 180
gaagggcatc atgcagagca aggaggtgaa gaagaaggaa aaaagcttcg agatgcacaa 240
gggcgaccag gacccccaga tcgccgccca cgtgattagc gaggccagca gcaagaccgc 300
cagcgtgctg cagtgggccc ccaagggcta ctacaccctg agcaccaacc tggtgaccct 360
ggagaacggc aggcagctgg ccgtgaagag gcagggcatc tactacatct acgcccaggt 420
gaccttctgc tccaacaggg acgccgctgg ccaggcccct ttcatcgcca gcctgtgcct 480
gaggagcccc agcggcagcg agcgcatcct gctgagggcc gccaacaccc acagcagcag 540
caagccctgt ggccagcaga gcatccacct gggcggcgtg ttcgagctgc agcctggcgc 600
cagcgtgttc gtgaacgtga ccgaccccag ccaggtgtcc cacggcaccg gcttcaccag 660
cttcggcctg ctgaagctgt gacccgggtc gac                              693

<210> 8
<211> 225
<212> PRT
<213> Sus scrofa

<220>
<221> PEPTIDE
<222> (1)..(225)
<223> Amino acid Sequence(staring by the "TCT" codon
     [nucleotide 5] of the Seq ID. No 7).

<400> 8
Ser Gly Gly Gly Ser Gly Gly Gly Gly Ser Gly Gly Gly Gly Ser Lys
  1               5                  10                  15

Ile Glu Asp Glu Arg Asn Leu His Glu Asp Phe Val Phe Ile Lys Thr
             20                  25                  30

Ile Gln Arg Cys Lys Gln Gly Glu Gly Ser Leu Ser Leu Leu Asn Cys
        35                  40                  45

Glu Glu Ile Arg Ser Gln Phe Glu Asp Leu Val Lys Gly Ile Met Gln
    50                  55                  60

Ser Lys Glu Val Lys Lys Lys Glu Lys Ser Phe Glu Met His Lys Gly
 65                  70                  75                  80

Asp Gln Asp Pro Gln Ile Ala Ala His Val Ile Ser Glu Ala Ser Ser
                 85                  90                  95

Lys Thr Ala Ser Val Leu Gln Trp Ala Pro Lys Gly Tyr Tyr Thr Leu
            100                 105                 110

Ser Thr Asn Leu Val Thr Leu Glu Asn Gly Arg Gln Leu Ala Val Lys

115                 120                 125

Arg Gln Gly Ile Tyr Tyr Ile Tyr Ala Gln Val Thr Phe Cys Ser Asn
    130                 135                 140

Arg Asp Ala Ala Gly Gln Ala Pro Phe Ile Ala Ser Leu Cys Leu Arg

145                 150                 155                 160

Ser Pro Ser Gly Ser Glu Arg Ile Leu Leu Arg Ala Ala Asn Thr His
                165                 170                 175

Ser Ser Ser Lys Pro Cys Gly Gln Gln Ser Ile His Leu Gly Gly Val
            180                 185                 190

Phe Glu Leu Gln Pro Gly Ala Ser Val Phe Val Asn Val Thr Asp Pro
        195                 200                 205

Ser Gln Val Ser His Gly Thr Gly Phe Thr Ser Phe Gly Leu Leu Lys
    210                 215                 220

Leu
225

<210> 9
<211> 536
<212> PRT
<213> Influenza A virus

<220>
<221> PEPTIDE
<222> (1)..(536)
<223> Sequence of the extracellular domains of the
      hemagglutinin of the Avian Influenza virus
      A/Netherlands/33/03(H7N7).

<400> 9
Met Asn Thr Gln Ile Leu Val Phe Ala Leu Val Ala Ile Ile Pro Thr
1               5                   10                  15

Asn Ala Asp Lys Ile Cys Leu Gly His His Ala Val Ser Asn Gly Thr
            20                  25                  30

Lys Val Asn Thr Leu Thr Glu Arg Gly Val Glu Val Val Asn Ala Thr
        35                  40                  45

Glu Thr Val Glu Arg Thr Asn Val Pro Arg Ile Cys Ser Lys Gly Lys
    50                  55                  60

Arg Thr Val Asp Leu Gly Gln Cys Gly Leu Leu Gly Thr Ile Thr Gly
65                  70                  75                  80

Pro Pro Gln Cys Asp Gln Phe Leu Glu Phe Ser Ala Asp Leu Ile Ile
                85                  90                  95

Glu Arg Arg Glu Gly Ser Asp Val Cys Tyr Pro Gly Lys Phe Val Asn
            100                 105                 110

Glu Glu Ala Leu Arg Gln Ile Leu Arg Glu Ser Gly Gly Ile Asp Lys
        115                 120                 125

Glu Thr Met Gly Phe Thr Tyr Ser Gly Ile Arg Thr Asn Gly Ala Thr
    130                 135                 140

Ser Ala Cys Arg Arg Ser Gly Ser Ser Phe Tyr Ala Glu Met Lys Trp
145                 150                 155                 160

Leu Leu Ser Asn Thr Asp Asn Ala Ala Phe Pro Gln Met Thr Lys Ser
                165                 170                 175

Tyr Lys Asn Thr Arg Lys Asp Pro Ala Leu Ile Ile Trp Gly Ile His
            180                 185                 190

His Ser Gly Ser Thr Thr Glu Gln Thr Lys Leu Tyr Gly Ser Gly Asn
        195                 200                 205

Lys Leu Ile Thr Val Gly Ser Ser Asn Tyr Gln Gln Ser Phe Val Pro
    210                 215                 220

Ser Pro Gly Ala Arg Pro Gln Val Asn Gly Gln Ser Gly Arg Ile Asp
225                 230                 235                 240

Phe His Trp Leu Ile Leu Asn Pro Asn Asp Thr Val Thr Phe Ser Phe
                245                 250                 255

Asn Gly Ala Phe Ile Ala Pro Asp Arg Ala Ser Phe Leu Arg Gly Lys
            260                 265                 270

Ser Met Gly Ile Gln Ser Glu Val Gln Val Asp Ala Asn Cys Glu Gly
        275                 280                 285

Asp Cys Tyr His Ser Gly Gly Thr Ile Ile Ser Asn Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Ile Pro
                325                 330                 335

Lys Arg Arg Arg Arg Gly Leu Phe Gly Ala Ile Ala Gly Phe Ile Glu
            340                 345                 350

Asn Gly Trp Glu Gly Leu Ile Asp Gly Trp Tyr Gly Phe Arg His Gln
        355                 360                 365

Asn Ala Gln Gly Glu Gly Thr Ala Ala Asp Tyr Lys Ser Thr Gln Ser
    370                 375                 380

Ala Ile Asp Gln Ile Thr Gly Lys Leu Asn Arg Leu Ile Glu Lys Thr
385                 390                 395                 400

Asn Gln Gln Phe Glu Leu Ile Asp Asn Glu Phe Thr Glu Val Glu Lys
                405                 410                 415

Gln Ile Gly Asn Val Ile Asn Trp Thr Arg Asp Ser Met Thr Glu Val
            420                 425                 430

Trp Ser Tyr Asn Ala Glu Leu Leu Val Ala Met Glu Asn Gln His Thr
        435                 440                 445

Ile Asp Leu Ala Asp Ser Glu Met Asn Lys Leu Tyr Glu Arg Val Lys
    450                 455                 460

Arg Gln Leu Arg Glu Asn Ala Glu Glu Asp Gly Thr Gly Cys Phe Glu
465                 470                 475                 480

Ile Phe His Lys Cys Asp Asp Asp Cys Met Ala Ser Ile Arg Asn Asn
                485                 490                 495

Thr Tyr Asp His Ser Lys Tyr Arg Glu Glu Ala Ile Gln Asn Arg Ile
            500                 505                 510

Gln Ile Asp Pro Val Lys Leu Ser Ser Gly Tyr Lys Asp Ala Ser His

515                    520                    525

His His His His His Met Asn Ser
    530                    535

<210> 10
<211> 537
<212> PRT
<213> Influenza A virus

<220>
<221> PEPTIDE
<222> (1)..(537)
<223> Sequence of the extracellular domains of the
      hemagglutinin of the Avian Influenza virus
      A/Hong Kong/1073/99 (H9N2).

<400> 10
Met Glu Thr Ile Ser Leu Ile Thr Ile Leu Leu Val Val Thr Ala Ser
1               5                   10                  15

Asn Ala Asp Lys Ile Cys Ile Gly His Gln Ser Thr Asn Ser Thr Glu
            20                  25                  30

Thr Val Asp Thr Leu Thr Glu Thr Asn Val Pro Val Thr His Ala Lys
        35                  40                  45

Glu Leu Leu His Thr Glu His Asn Gly Met Leu Cys Ala Thr Ser Leu
    50                  55                  60

Gly His Pro Leu Ile Leu Asp Thr Cys Thr Ile Glu Gly Leu Val Tyr
65                  70                  75                  80

Gly Asn Pro Ser Cys Asp Leu Leu Leu Gly Gly Arg Glu Trp Ser Tyr
                85                  90                  95

Ile Val Glu Arg Ser Ser Ala Val Asn Gly Thr Cys Tyr Pro Gly Asn
            100                 105                 110

Val Glu Asn Leu Glu Glu Leu Arg Thr Leu Phe Ser Ser Ala Ser Ser
        115                 120                 125

Tyr Gln Arg Ile Gln Ile Phe Pro Asp Thr Thr Trp Asn Val Thr Tyr
    130                 135                 140

Thr Gly Thr Ser Arg Ala Cys Ser Gly Ser Phe Tyr Arg Ser Met Arg
145                 150                 155                 160

Trp Leu Thr Gln Lys Ser Gly Phe Tyr Pro Val Gln Asp Ala Gln Tyr
                165                 170                 175

Thr Asn Asn Arg Gly Lys Ser Ile Leu Phe Val Trp Gly Ile His His
            180                 185                 190

Pro Pro Thr Tyr Thr Glu Gln Thr Asn Leu Tyr Ile Arg Asn Asp Thr
        195                 200                 205

Thr Thr Ser Val Thr Thr Glu Asp Leu Asn Arg Thr Phe Lys Pro Val
    210                 215                 220

Ile Gly Pro Arg Pro Leu Val Asn Gly Leu Gln Gly Arg Ile Asp Tyr
225                 230                 235                 240

Tyr Trp Ser Val Leu Lys Pro Gly Gln Thr Leu Arg Val Arg Ser Asn
                245                 250                 255

Gly Asn Leu Ile Ala Pro Trp Tyr Gly His Val Leu Ser Gly Gly Ser

            260                 265                 270

His Gly Arg Ile Leu Lys Thr Asp Leu Lys Gly Gly Asn Cys Val Val
        275                 280                 285

Gln Cys Gln Thr Glu Lys Gly Gly Leu Asn Ser Thr Leu Pro Phe His
    290                 295                 300

Asn Ile Ser Lys Tyr Ala Phe Gly Thr Cys Pro Lys Tyr Val Arg Val
305                 310                 315                 320

Asn Ser Leu Lys Leu Ala Val Gly Leu Arg Asn Val Pro Ala Arg Ser
                325                 330                 335

Ser Arg Gly Leu Phe Gly Ala Ile Ala Gly Phe Ile Glu Gly Gly Trp
            340                 345                 350

Pro Gly Leu Val Ala Gly Trp Tyr Gly Phe Gln His Ser Asn Asp Gln
        355                 360                 365

Gly Val Gly Met Ala Ala Asp Arg Asp Ser Thr Gln Lys Ala Ile Asp
    370                 375                 380

Lys Ile Thr Ser Lys Val Asn Asn Ile Val Asp Lys Met Asn Lys Gln
385                 390                 395                 400

Tyr Glu Ile Ile Asp His Glu Phe Ser Glu Val Glu Thr Arg Leu Asn
                405                 410                 415

Met Ile Asn Asn Lys Ile Asp Asp Gln Ile Gln Asp Val Trp Ala Tyr
            420                 425                 430

Asn Ala Glu Leu Leu Val Leu Leu Glu Asn Gln Lys Thr Leu Asp Glu
        435                 440                 445

His Asp Ala Asn Val Asn Asn Leu Tyr Asn Lys Val Lys Arg Ala Leu
    450                 455                 460

Gly Ser Asn Ala Met Glu Asp Gly Lys Gly Cys Phe Glu Leu Tyr His
465                 470                 475                 480

Lys Cys Asp Asp Gln Cys Met Glu Thr Ile Arg Asn Gly Thr Tyr Asn
                485                 490                 495

Arg Arg Lys Tyr Arg Glu Glu Ser Arg Leu Glu Arg Gln Lys Ile Glu
            500                 505                 510

Gly Val Lys Leu Glu Ser Glu Gly Thr Tyr Lys Ile Leu Thr Ala Ser
        515                 520                 525

His His His His His His Met Asn Ser
    530                 535

## Claims

1. Chimeric vaccine antigen against Avian Influenza virus, **characterized** for containing the extracellular segment of the HA protein from the avian influenza virus envelope and the extracellular segment of the CD154 molecule.

2. Chimeric vaccine antigen according to claim 1, **characterized** for containing essentially the amino acid sequences of the extracellular segment of the CD154 molecule from avian (Seq ID. No. 6), swine (Seq ID. No. 8) or human (Seq ID. No. 4).

3. Chimeric vaccine antigen according to claim 1, **characterized** for containing essentially the amino acid sequences of the extracellular segment of HA from the virus subtypes H5 (Seq ID. No. 2), H7 (Seq ID. No.9) and H9 (Seq ID. No.10).

4. Chimeric vaccine antigen according to claim 1, obtained by recombinant way, synthetic way or through chemical conjugation.

5. Chimeric vaccine antigen according to claim 1, obtained from the milk of genetically modified mammalians.

6. Chimeric vaccine antigen according to claim 5, obtained from the milk of non transgenic mammalians, by direct genetic transformation of the mammary gland.

7. Chimeric vaccine antigen according to claim 6, where direct genetic transformation of the mammary gland is made by employing adenoviral vector transduction.

8. Chimeric vaccine antigen according to claim 5, obtained from the milk of transgenic mammalians.

9. Chimeric vaccine antigen according to claim 4, obtained from genetically modified yeasts.

10. Vaccine composition able to produce a protective immune response against Avian Influenza virus in birds and mammalians, **characterized by** containing the chimeric antigens described on claims 1-9.

11. Vaccine composition according to claim 10, able to produce a protective immune response against Avian Influenza virus in birds, pigs and humans.

12. Vaccine composition according to claim 10, which can be preventively administered to animals, by systemic or mucosal route.
